(19) Europäisches Patentamt
European Patent Office
Office européen des brevets

(11) Veröffentlichungsnummer: **0 398 171 A2**

(12) **EUROPÄISCHE PATENTANMELDUNG**

(21) Anmeldenummer: **90108874.0**

(51) Int. Cl.5: **A61K 31/55**

(22) Anmeldetag: **11.05.90**

(30) Priorität: **19.05.89 CH 1886/89**

(43) Veröffentlichungstag der Anmeldung: **22.11.90 Patentblatt 90/47**

(84) Benannte Vertragsstaaten: **AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Scollo-Lavizzari, Giuseppe, Prof.-Dr.**
**Hirzbodenweg 96**
**CH-4052 Basel(CH)**

(74) Vertreter: **Lederer, Franz, Dr. et al**
**Lederer, Keller & Riederer, Patentanwälte,**
**Lucile-Grahn-Strasse 22**
**D-8000 München 80(DE)**

(54) **Verwendung von Imidazodiazepinen bei der Behandlung von neurologischen Symptomen, welche mit zirkulatorischen Störungen des Gehirns assoziiert sind.**

(57) Die bekannten Verbindungen der allgemeinen Formel

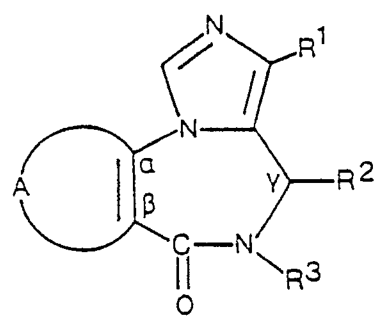

I

worin A zusammen mit den beiden mit α und β bezeichneten Kohlenstoffatomen die Gruppe

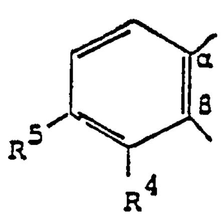

(a)

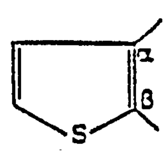

(b) oder

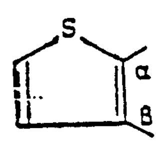

(c),

$R^1$ Halogen, Cyano, niederes Alkyl, niederes 1-Alkenyl, niederes Alkoxymethyl oder die Gruppe -COOR$^6$ oder -C≡C-R$^7$, $R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl, $R^6$ niederes Alkyl, $C_{3-7}$-Cycloalkyl oder $C_{3-7}$-Cycloalkyl-niederes Alkyl und $R^7$ Wasserstoff, niederes Alkyl, niederes Hydroxy-

EP 0 398 171 A2

alkyl, niederes Alkoxyalkyl, $C_{3-7}$-Cycloalkyl-niederes Alkyl, $C_{3-7}$-Cycloalkyl-niederes Hydroxyalkyl, $C_{3-7}$-Cycloalkyl-niederes Alkoxyalkyl, ($C_{3-7}$-Cycloalkyl-niederes Alkoxy)-niederes Alkyl, (Aryl-niederes Alkoxy)-niederes Alkyl, niederes Alkanoyloxy-niederes Alkyl, ($C_{3-7}$-Cycloalkyl-niederes Alkanoyloxy)-niederes Alkyl, $C_{3-7}$-Cycloalkylcarbonyloxy-niederes Alkyl, (Arylniederes Alkanoyloxy)-niederes Alkyl, Arylcarbonyloxy-niederes Alkyl, niederes Alkenyl, niederes Hydroxyalkenyl, niederes Alkoxy-niederes Alkenyl, ($C_{3-7}$- Cycloalkylniederes Alkoxy)-niederes Alkenyl, (Aryl-niederes Alkoxy)-niederes Alkenyl, niederes Alkanoyloxy-niederes Alkenyl, ($C_{3-7}$-Cycloalkyl-niederes Alkanoyloxy)-niederes Alkenyl, $C_{3-7}$-Cycloalkylcarbonyloxy-niederes Alkenyl, (Aryl-niederes Alkanoyloxy)-niederes Alkenyl, Arylcarbonyloxy-niederes Alkenyl, $C_{3-7}$-Cycloalkyl, Hydroxy-$C_{4-7}$-Cycloalkyl oder niederes Alkoxy-$C_{4-7}$-Cycloalkyl bedeuten, wobei die Verbindungen der Formel I bezüglich des mit Y bezeichneten Kohlenstoffatoms die (S)- oder (R,S)-Konfiguration aufweisen, wenn $R^2$ und $R^3$ zusammen Dimethylen oder Trimethylen bedeuten,
können bei der Behandlung von neurologischen Symptomen, welche mit zirkulatorischen Störungen des Gehirns assoziiert sind, verwendet werden.

## Verwendung von Imidazodiazepinen bei der Behandlung von neurologischen Symptomen, welche mit zirkulatorischen Störungen des Gehirns assoziiert sind

Die vorliegende Erfindung betrifft die Verwendung von Verbindungen der allgemeinen Formel

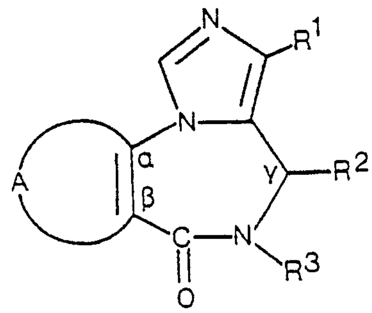

I

worin A zusammen mit den beiden mit α und β bezeichneten Kohlenstoffatomen die Gruppe

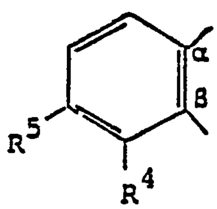 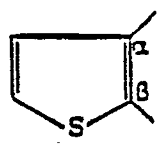 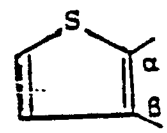

(a) (b) oder (c),

$R^1$ Halogen, Cyano, niederes Alkyl, niederes 1-Alkenyl, niederes Alkoxymethyl oder die Gruppe $-COOR^6$ oder $-C{\equiv}C-R^7$, $R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl, $R^6$ niederes Alkyl, $C_{3-7}$-Cycloalkyl oder $C_{3-7}$-Cycloalkyl-niederes Alkyl und $R^7$ Wasserstoff, niederes Alkyl, niederes Hydroxyalkyl, niederes Alkoxyalkyl, $C_{3-7}$-Cycloalkyl-niederes Alkyl, $C_{3-7}$-Cycloalkyl-niederes Hydroxyalkyl, $C_{3-7}$-Cycloalkyl-niederes Alkoxyalkyl, ($C_{3-7}$-Cycloalkyl-niederes Alkoxy)-niederes Alkyl, (Aryl-niederes Alkoxy)-niederes Alkyl, niederes Alkanoyloxy-niederes Alkyl, ($C_{3-7}$-Cycloalkyl-niederes Alkanoyloxy)-niederes Alkyl, $C_{3-7}$-Cycloalkylcarbonyloxy-niederes Alkyl, (Arylniederes Alkanoyloxy)-niederes Alkyl, Arylcarbonyloxy-niederes Alkyl, niederes Alkenyl, niederes Hydroxyalkenyl, niederes Alkoxy-niederes Alkenyl, ($C_{3-7}$-Cycloalkyl-niederes Alkoxy)-niederes Alkenyl, (Aryl-niederes Alkoxy)-niederes Alkenyl, niederes Alkanoyloxy-niederes Alkenyl, ($C_{3-7}$-Cycloalkyl-niederes Alkanoyloxy)-niederes Alkenyl, $C_{3-7}$-Cycloalkylcarbonyloxy-niederes Alkenyl, (Aryl-niederes Alkanoyloxy)-niederes Alkenyl, Arylcarbonyloxy-niederes Alkenyl, $C_{3-7}$-Cycloalkyl, Hydroxy-$C_{4-7}$-Cycloalkyl oder niederes Alkoxy-$C_{4-7}$-Cycloalkyl bedeuten, wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten,
bei der Behandlung von neurologischen Symptomen, welche mit zirklatorischen Störungen des Gehirns assoziiert sind, insbesonders bei der Behandlung von neurologischen Symptomen, welche mit cerebrovaskulären Insulten assoziiert sind.

Gegenstand der vorliegenden Erfindung sind: Die Verwendung von Verbindungen der obigen Formel I bei der Behandlung von neurologischen Symptomen, welche mit zirkulatorischen Störungen des Gehirns assoziiert sind, die Verwendung von Verbindungen der obigen Formel I zur Herstellung von entsprechenden Mitteln, sowie ein Verfahren und Mittel zur Behandlung der erwähnten neurologischen Symptome.

Der Ausdruck "nieder" wird verwendet, um Reste und Verbindungen mit bis zu 7, vorzugsweise bis zu 4 Kohlenstoffatomen zu bezeichnen. Der Ausdruck "Alkyl" bezeichnet geradkettige oder verzweigte gesättigte Kohlenwasserstoffreste wie Methyl, Aethyl, n-Propyl, Isopropyl, n-Butyl, Isobutyl, s-Butyl, t-Butyl und dergleichen. Der Ausdruck "Alkoxy" bezeichnet über ein Sauerstoffatom gebundene Alkylreste im Sinne der vorstehenden Definition des Ausdruckes "Alkyl". Der Ausdruck "Cycloalkyl" bezeichnet cyclische, gesättigte Kohlenwasserstoffreste, wie Cyclopropyl, Cyclobutyl, Cyclopentyl und Cyclohexyl. Der Ausdruck

"Alkanoyloxy" bezeichnet von geradkettigen oder verzweigten Fettsäuren abgeleitete Reste, wie Acetoxy. Der Ausdruck "Alkenyl" bezeichnet geradkettige oder verzweigte Kohlenwasserstoffreste, welche mindestens eine olefinische Doppelbindung enthalten, wie cis- und trans-2-Buten-2-yl und 1-Buten-3-yl. Der Ausdruck "Aryl" bezeichnet vorzugsweise monocyclische aromatische Kohlenwasserstoffreste, welche vorzugsweise unsubstituiert oder durch niederes Alkyl, niederes Alkoxy und/oder Halogen substituiert sind. Der Ausdruck "Halogen" bezeichnet die vier Halogene Fluor, Chlor, Brom und Jod.

Die Verbindungen der obigen Formel I sind bekannte Substanzen, ihre Herstellung und ihre bekannten benzodiazepinantagonistischen Eigenschaften werden beispielsweise in den Europäischen Patentpublikationen Nr. 27 214, 59 387, 59 389, 59 390, 100 906 und 285 837 beschrieben.

Im Rahmen der vorliegenden Erfindung wird als Verbindung der Formel I vorzugsweise Flumazenil, Aethyl-8-fluor-6-oxo-4H-imidazo[1,5-a][1,4]benzodiazepin-3-carboxylat, verwendet.

Auf Grund der benzodiazepinantagonistischen Eigenschaften sind die Verbindungen der obigen Formel I in der Lage, die zentralen Effekte von Präparaten, die ihre Wirkung über die Benzodiazepinrezeptoren entfalten,durch kompetitive Hemmung spezifisch zu blockieren. Sie können daher für die Aufhebung der zentral dämpfenden Wirkungen von Benzodiazepinen verwenden werden. In der Anästhesie können sie beispielsweise für die Beendigung der durch Benzodiazepine eingeleiteten und aufrechterhaltenen Narkose bei stationären Patienten verwendet werden. In der Intensivpflege können sie beispielsweise für die Aufhebung der zentralen Effekte von Benzodiazepinen bei Arzneimittelüberdosierung verwendet werden, um Spontanatmung und Bewusstsein wieder herbeizuführen.

Es wurde nun überraschenderweise gefunden, dass sie ebenfalls wirksam sind bei der Behandlung von neurologischen Symptomen, welche mit zirkulatorischen Störungen des Gehirns assoziiert sind, d.h. in keinem Zusammenhang mit der Verabreichung oder Einnahme von Benzodiazepinen stehen.

Zirkulatorische Störungen des Gehirns, wie sie beispielsweise bei cerebrovaskulären Insulten auftreten, sind gekennzeichnet durch eine plötzlich oder subakut auftretende fokale Symptomatik. Typische neurologische Symptome sind Lähmungen, Sensibilitätsstörungen, Bewusstseinsstörungen, Sprachstörungen und andere Kommunikationsstörungen.

Erfindungsgemäss können die Verbindungen der obigen Formel I verwendet werden, um neurologische Symptome nach zirkulatorischen Störungen des Gehirns, einschiesslich der oben erwähnten, zu behandeln. Beispielsweise kann das Bewusstsein von stuporotischen oder komatösen Patienten nach cerebrovaskulärem Insult wieder herbeigeführt werden. Die Patienten sind nach der Behandlung mit einer Verbindung der Formel I in der Regel sofort ansprechbar und reagieren auf einfache Aufforderungen. Dieser Befund ist in Einklang mit den aufgenommenen Elektroencephalogrammen und den periodisch mittels piezoelektrischer Sensoren (Actigraphen) gemessenen und quantifizierten motorischen Aktivitäten der Hände.

Bei hemiplegischen Patienten konnte nach der Behandlung mit einer Verbindung der Formel I eine signifikante Rückbildung der Paresen beobachtet werden.

Es stehen derzeit keine Arzneimittel zur Verfügung, um neurologische Symptome bei Patienten mit zirkulatorischen Störungen des Gehirns zu behandeln. Die mit Verbindungen der obigen Formel I gegebene Möglichkeit, derartige Symptome zu behandeln, eröffnet offensichtlich grosse Vorteile bei der Diagnose, der Therapie und der Rehabilitation. Die Aufhebung der Bewusstseinsstörungen ist beispielsweise entscheidend für die Prognose. Die Entwicklung von bleibenden neurologischen Störungen kann frühzeitig verhindert oder zumindest stark gehemmt werden. Die Rehabilitation wird erleichtert und allfällige physiotherapeutische Massnahmen können früher beginnen. Diese durch die erfindungsgemässe Verwendung von Verbindungen der obigen Formel I eröffneten Möglichkeiten dürften die Heilungsaussichten der Patienten wesentlich verbessern.

Die erfindungsgemässe Wirkung der Verbindungen der obigen Formel I kann anhand der fünf nachfolgend beschriebenen Fälle gezeigt werden, bei welchen Flumazenil als repräsentativer Vertreter der durch die Formel I definierten Verbindungsklasse verwendet worden ist.

Die Patienten Nos. 1 - 3, welche in die Studie miteinbezogen wurden, wurden mit Symptomen und dem Verdacht eines cerebrovaskulären Insultes, assoziiert mit Störungen des Bewusstseins, hospitalisiert. Alle drei Patienten hatten keine Benzodiazepine eingenommen, was im Verlauf der Studie durch ein negatives Resultat im Benzodiazepin-Screening bestätigt worden ist. Die Patienten befanden sich in einem stabilen hämodynamischen und respiratorischen Zustand, ohne Elektrolytenabnormalität oder anderen Ursachen für die neurologischen Symptome .

Vor Verabreichung von Flumazenil und während zwei Stunden danach wurde eine vollständige neurologische Untersuchung durchgeführt, ebenso wurden die hämodynamischen und respiratorischen Funktionen überwacht, und zwei Patienten wurden elektroencephalographisch überwacht. Beim dritten Patienten wurde ein Actigraph zur Messung und Quantifizierung der motorischen Aktivität der Hände verwendet.

Patient Nr. 1

Eine 72 Jahre alte Frau wurde mit der Diagnose eines cerebrovaskulären Insultes, assoziiert mit rechter Hemiplegie und Stupor, eingeliefert. Computertomographie deutete auf multiple Infarkte ohne Anzeichen einer interkranialen Blutung.

Nach intravenöser Verabreichung von 2 mg Flumazenil während 15 Minuten antwortete die Patientin auf einfache Befehle. Im Verlauf der nächsten Stunde verlor sie wieder ihre Bewusstsein, worauf sie weitere 1 mg Flumazenil während 10 Minuten injiziert erhielt und dann wieder auf einfache Befehle antwortete. Das Rest-EEG zeigte Asymmetrie und praktisch keine $\alpha$-Aktivität. Nach 10 Minuten änderte das Muster auf $\alpha$-Aktivität auf der rechten Seite.

Patient Nr. 2

Ein 52 Jahre alter Mann, ein regelmässiger Dialysepatient, wurde in einem komatösen Zustand eingeliefert. Der Patient befand sich im tiefen Koma und zeigte keine fokalen neurologischen Zeichen. Fundoskopie war normal. Die Blutanalyse zeigte eine metabolische Azidose mit einem pH von 7,25 mit einer Hyperkalämie von 6,2 mval/l.

Wegen vermuteter Einnahme von mehr als einem Arzneimittel einschliesslich Benzodiazepinen wurde eine Notfalldialyse durchgeführt. Nach vier Stunden hatte sich der Zustand des Patienten nicht verbessert. Er erhielt dann 1 mg Flumazenil über einen Zeitraum von 10 Minuten injiziert, worauf der Patient das volle Bewusstsein wieder erlangte und kategorisch bestritt, irgendwelche Arzneimittel eingenommen zu haben. Die toxikologische Untersuchung bestätigte diese Aussage; es konnten keine Blutspiegel an Barbituraten oder Benzodiazepinen nachgewiesen werden. Die später durchgeführte computertomographische Untersuchung ergab kortikale und zentral-generalisierte Atrophie, lakunäre Infarkte im rechten Putamen und im linken kaudalen Kern und Kalkablagerungen im Raume des Tentorums und des Pallidums. Der Patient konnte später mit normalem neurologischem Befund und ohne Folgeerscheinungen entlassen werden.

Patient Nr. 3

Bei einer 89 Jahre alten Frau traten rechte Hemiplegie und Stupor auf. 10 Minuten nach Verabreichung von 1 mg Flumazenil wachte sie auf und reagierte auf verbale Befehle, wobei sie auch leichte Bewegungen ihrer gelähmten Seite des Körpers zeigte.

Mit dem Actigraph konnte ein statistisch signifikanter Unterschied zwischen den Bewegungen vor und nach der Behandlung festgestellt werden. Die Patientin konnte später bei vollem Bewusstsein und mit einer geringfügigen motorischen Schwäche entlassen werden.

Die Patienten Nos. 4 und 5, welche in die Studie miteinbezogen wurden, wurden ebenfalls mit Symptomen und dem Verdacht eines cerebrovaskulären Insultes hospitalisiert. Beide waren hemiplegisch, ansprechbar und kooperativ. Bei beiden Patienten wurde die Entwicklung der Paresen untersucht, indem die Kraft der Hände vor und nach der Behandlung mit Flumazenil gemessen wurde.

Patient Nr. 4

Eine 42 Jahre alte Frau wurde mit der Diagnose eines cerebrovaskulären Insultes im Bereich der Capsula externa rechts mit weitgehend passagerer Hemisymptomatik links und Migräne hospitalisiert.

Der cerebrovaskuläre Insult traf aus völligem Wohlbefinden heraus auf. Die Patientin hatte vorher keine Kopfschmerzen, und es bestanden keine Risikofaktoren. 11 Tage nach der Hospitalisierung ergab die Kraftmessung der Hände rechts 7 kg und links 3 kg. Anschliessend erhielt die Patientin 10 mg Flumazenil peroral. Die 10 Minuten später durchgeführte Kraftmessung ergab rechts 7,5 kg und links 7 kg. Nach 20 Minuten betrug die Kraft rechts 8 kg und links 6 kg, nach 30 Minuten rechts 7,8 kg und links 6 kg. Die Behandlung mit Flumazenil bewirkte eine signifikante Zunahme der Kraft links und damit eine deutliche Rückbildung der Parese.

Aufgrund der durchgeführten Abklärungen konnte eine hämodynamische Behinderung im Bereich der cerebralen Gefässe ausgeschlossen werden; eine Emboliequelle konnte dort sowie im Bereich des Herzens nicht nachgewiesen werden. Zwei Tage nach der Behandlung mit Flumazenil war die Patientin völlig erholt und konnte mit nur noch diskreten neurologischen Ausfällen nach Hause entlassen werden.

Patient Nr. 5

Nach dem Aufstehen verspürte die Patientin, eine 47 Jahre alte Frau, aus völligem Wohlsein heraus plötzlich einen akuten Drehschwindel sowie ein Leeregefühl im Kopf. Sie hat nicht mehr aufstehen können. Etwas später traten handbetonte Kribbelparästhesien im Bereich der gesamten rechten Körperhälfte auf, und etwas später auch eine Kraftlosigkeit der rechten Hand. Kurzzeitig hatte sie auch etwas Mühe mit dem Sprechen.

Nach der Hospitalisierung wurde ein akuter cerebrovaskulärer Insult im Bereich der Capsula interna links mit weitgehend passageren sensomotorischen Ausfällen im Bereich der rechten Körperhälfte diagnostiziert. Es konnte ein solitärer Infarkt nachgewiesen werden. Eine demyelinisierende Erkrankung und eine Vaskulitis konnten aufgrund der erhobenen Befunde mit an Sicherheit grenzender Wahrscheinlichkeit ausgeschlossen werden. Drei Tage nach der Hospitalisierung ergab die Messung der Kraft der Hände links 8 kg und rechts 5 kg. Die Patientin erhielt dann 10 mg Flumazenil peroral. Nach 15 und 30 Minuten wurde die Kraftmessung wiederholt und ergab jeweils beidseits je 8 kg. Kurze Zeit nach der Behandlung mit Flumazenil gab die Patientin subjektiv eine Verminderung der Parästhesien der rechten Hand an.

Die Patientin konnte 5 Tage später entlassen werden. Die motorischen Ausfälle waren praktisch vollständig verschwunden. Die Behandlung mit Flumazenil bewirkte eine signifikante Besserung bei der Kraftprüfung und auch eine subjektive Besserung bezüglich der Sensibilitätsstörungen (Verminderung der Parästhesien der rechten Hand).

Im Rahmen der vorliegenden Erfindung können die Verbindungen der obigen Formel I in Form von peroral, rektal oder parenteral verabreichbaren pharmazeutischen Präparaten verwendet werden, z.B. in Form von Tabletten, Lacktabletten, Dragees, Hart- und Weichgelatinekapseln, Suppositorien oder Lösungen. Sie können auch in Form von Infusionen verabreicht werden. Als Dosierungsform werden Tabletten zur peroralen und Injektionslösungen und Infusionslösungen zur intravenösen Verabreichung bevorzugt.

Zur Herstellung von pharmazeutischen Präparaten werden die Verbindungen der obigen Formel I mit pharmazeutisch inerten, anorganischen oder organischen Trägern verarbeitet. Als solche Träger kann man für Tabletten, Lacktabletten, Dragees und Gelatinekapseln beispielsweise Milchzucker, Maisstärke oder Derivate davon, Talk, Stearinsäure oder deren Salze und dergleichen verwenden. Für Suppositorien eignen sich als Träger beispielsweise natürliche oder gehärtete Oele, Wachse, Fette, halbfeste und flüssige Polyole und dergleichen. Zur Herstellung von Lösungen eignen sich als Träger beispielsweise Wasser, Polyole, Saccharose, Invertzucker, Glukose und dergleichen.

Die pharmazeutischen Präparate könne daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Wie eingangs erwähnt, können die Verbindungen der obigen Formel I bei der Behandlung von neurologischen Symptomen, welche mit zirkulatorischen Störungen des Gehirns assoziiert sind, verwendet werden. Die Dosierung kann je nach Schwere der Erkrankung, Alter und Gewicht des Patienten variieren und ist natürlich in jedem einzelnen Fall den individuellen Gegebenheiten anzupassen. Im allgemeinen dürfte bei peroraler Verabreichung eine Dosis von 5 mg bis 50 mg, vorzugsweise 10 mg bis 30 mg, und bei intravenöser Verabreichung eine Dosis von etwa 0,5 mg bis 5 mg, vorzugsweise 1 mg bis 3 mg angemessen sein, wobei diese Dosierungen auch aufgeteilt und/oder wiederholt appliziert werden können.

Die nachfolgenden Beispiele beschreiben für die praktische Anwendung der vorliegenden Erfindung geeignete Dosierungsformen. Sie sollen ihren Umfang jedoch in keiner Weise beschränken.

## Beispiel 1

Es werden Injektionslösungen folgender Zusammensetzung in an sich bekannter Weise hergestellt:

| | | |
|---|---|---|
| Flumazenil | 0,5 mg | 1 mg |
| Wasser zu Injektionszwecken, enthaltend Aethylendiamintetraessigsäure, Essigsäure und Natriumchlorid als Hilfsstoffe | 5 ml | 10 ml |

Infusionslösungen können hergestellt werden, indem man die obigen Injektionslösungen mit 0,9-proz. Natriumchlorid, 0,45-proz. Natriumchlorid/2,5-proz. Dextrose oder mit 5-proz. Dextrose für Infusionszwecke verdünnt.

Beispiel 2

Es werden Tabletten folgender Zusammensetzung in an sich bekannter Weise hergestellt:

| | | mg/Tablette |
|---|---|---|
| Flumazenil | | 10 |
| Milchzucker | | 90 |
| Maisstärke | | 29 |
| Mikrokristalline Cellulose | | 70 |
| Magnesiumstearat | | 1 |
| | Total | 200 mg |

Beispiel 3

Es werden Kapseln folgender Zusammensetzung in an sich bekannter Weise hergestellt:

| | | mg/Kapseln |
|---|---|---|
| Flumazenil | | 10 |
| Milchzucker | | 165 |
| Maisstärke | | 30 |
| Talk | | 5 |
| | Total | 210 mg |

**Ansprüche**

1. Verwendung von Verbindungen der allgemeinen Formel

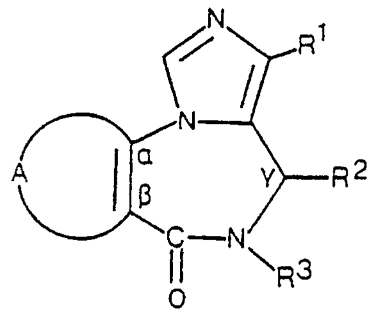

I

worin A zusammen mit den beiden mit α und β bezeichneten Kohlenstoffatomen die Gruppe

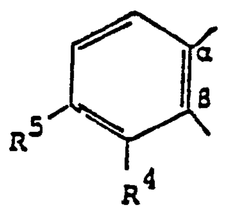

(a)

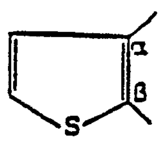

(b) oder

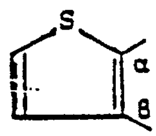

(c),

worin $R^1$ Halogen, Cyano, niederes Alkyl, niederes 1-Alkenyl, niederes Alkoxymethyl oder die Gruppe $-COOR^6$ oder $-C{\equiv}C-R^7$, $R^2$ Wasserstoff und $R^3$ niederes Alkyl oder $R^2$ und $R^3$ zusammen Dimethylen oder Trimethylen, $R^4$ und $R^5$ je Wasserstoff, Halogen, Trifluormethyl oder niederes Alkyl, $R^6$ niederes Alkyl, $C_{3-7}$-Cycloalkyl oder $C_{3-7}$-Cycloalkyl-niederes Alkyl und $R^7$ Wasserstoff, niederes Alkyl, niederes Hydroxyalkyl, niederes Alkoxyalkyl, $C_{3-7}$-Cycloalkyl-niederes Alkyl, $C_{3-7}$-Cycloalkyl-niederes Hydroxyalkyl, $C_{3-7}$-Cycloalkyl-niederes Alkoxyalkyl, ($C_{3-7}$-Cycloalkyl-niederes Alkoxy)-niederes Alkyl, (Aryl-niederes Alkoxy)-niederes Alkyl, niederes Alkanoyloxy-niederes Alkyl, ($C_{3-7}$-Cycloalkyl-niederes Alkanoyloxy)-niederes Alkyl, $C_{3-7}$-Cycloalkylcarbonyloxy-niederes Alkyl, (Aryl-niederes Alkanoyloxy)-niederes Alkyl, Arylcarbonyloxy-niederes Alkyl, niederes Alkenyl, niederes Hydroxyalkenyl, niederes Alkoxy-niederes Alkenyl, ($C_{3-7}$-Cycloalkyl-niederes Alkoxy)-niederes Alkenyl, (Aryl-niederes Alkoxy)-niederes Alkenyl, niederes Alkanoyloxy-niederes Alkenyl, ($C_{3-7}$-Cycloalkyl-niederes Alkanoyloxy)-niederes Alkenyl, $C_{3-7}$-Cycloalkylcarbonyloxy-niederes Alkenyl, (Aryl-niederes Alkanoyloxy)-niederes Alkenyl, Arylcarbonyloxy-niederes Alkenyl, $C_{3-7}$-Cycloalkyl, Hydroxy-$C_{4-7}$-Cycloalkyl oder niederes Alkoxy-$C_{4-7}$-Cycloalkyl bedeuten, wobei die Verbindungen der Formel I bezüglich des mit γ bezeichneten Kohlenstoffatoms die (S)- oder (R,S)-Konfiguration aufweisen, wenn $R^2$ und $R^3$ zusammen Dimethylen oder Trimethylen bedeuten, zur Herstellung von Mitteln zur Behandlung von neurologischen Symptomen, welche mit zirkulatorischen Störungen des Gehirns assoziiert sind.

2. Verwendung von Aethyl 8-fluor-5,6-dihydro-6-oxo-4H-imidazo[1,5-a][1,4] benzodiazepin-3-carboxylat zur Herstellung von Mitteln zur Behandlung von neurologischen Symptomen, welche mit zirkulatorischen Störungen des Gehirns assoziiert sind.

3. Verfahren zur Herstellung einer Arzneimittelpackung, enthaltend ein Mittel auf der Basis einer Verbindung der in Anspruch 1 definierten Formel I, dadurch gekennzeichnet, dass man eine Arzneimittelpackung für die Behandlung von neurologischen Symptomen, welche mit zirkulatorischen Störungen des Gehirns assoziiert sind, herstellt, bestehend aus der Kombination eines Mittels auf der Basis einer Verbindung der in Anspruch 1 definierten Formel I zusammen mit der Information, welche in direktem Zusammenhang mit der Behandlung von neurologischen Symptomen, welche mit zirkulatorischen Störungen des Gehirns assoziiert sind, steht.

4. Verfahren zur Herstellung einer Arzneimittelpackung, enthaltend ein Mittel auf der Basis von Aethyl 8-fluor-5,6-dihydro-6-oxo-4H-imidazo[1,5-a][1,4]benzodiazepin-3-carboxylat, dadurch gekennzeichnet, dass man eine Arzneimittelpackung für die Behandlung von neurologischen Symptomen, welche mit zirkulatorischen Störungen des Gehirns assoziiert sind, herstellt, bestehend aus der Kombination eines Mittels auf der Basis von Aethyl 8-fluor-5,6-dihydro-6-oxo-4H-imidazo[1,5-a][1,4]benzodiazepin-3-carboxylat zusammen mit der Information, welche in direktem Zusammenhang mit der Behandlung von neurologischen Symptomen, welche mit zirkulatorischen Störungen des Gehirns assoziiert sind, steht.

5. Verfahren zur Herstellung von Arzneimitteln, welche als Wirkstoff eine Verbindung der in Anspruch 1 definierten Formel I sowie übliche Trägermaterialien enthalten, dadurch gekennzeichnet, dass man den auf bekannte Weise hergestellten Wirkstoff mit üblichen Trägermaterialien vermischt und in ein Arzneimittel zur Behandlung von neurologischen Symptomen, welche mit zirkulatorischen Störungen des Gehirns assoziiert sind, überführt.

6. Verfahren zur Herstellung von Arzneimitteln, welche als Wirkstoff Aethyl 8-fluor-5,6-dihydro-6-oxo-4H-imidazo[1,5-a][1,4]benzodiazepin-3-carboxylat sowie übliche Trägermaterialien enthalten, dadurch gekennzeichnet, dass man den auf bekannte Weise hergestellten Wirkstoff mit üblichen Trägermaterialien vermischt und in ein Arzneimittel zur Behandlung von neurologischen Symptomen, welche mit zirkulatorischen Störungen des Gehirns assoziiert sind, überführt.

7. Arzneimittelpackung enthaltend ein Mittel auf der Basis einer Verbindung der in Anspruch 1 definierten Formel I zusammen mit Instruktionen für dessen Verwendbarkeit zur Behandlung von neurologischen Symptomen, welche mit zirkulatorischen Störungen des Gehirns assoziiert sind.

8. Arzneimittelpackung enthaltend ein Mittel auf der Basis von Aethyl 8-fluor-5,6-dihydro-6-oxo-4H-imidazo[1,5-a][1,4]benzodiazepin-3-carboxylat zusammen mit Instruktionen für dessen Verwendbarkeit zur Behandlung von neurologischen Symptomen, welche mit zirkulatorischen Störungen des Gehirns assoziiert sind.